# EUROPÄISCHE PATENTSCHRIFT

(11) **EP 0 909 761 B1**
(45) Veröffentlichungstag und Bekanntmachung des Hinweises auf die Patenterteilung: **02.04.2003**
(21) Anmeldenummer: 98250336.9
(22) Anmeldetag: 22.09.1998
(51) Int. Cl.: C07F 9/38, A61K 6/00, C08F 30/02

(54) **Hydrolysestabile und polymerisierbare Acrylphosphonsäuren**
Hydrolysis-stable and polymerizable acryl phosphonic acids
Acides acryl phosphoniques stables à l'hydrolyse et polymérisables

(30) Priorität: 16.10.1997 DE 19746708
(43) Veröffentlichungstag der Anmeldung: 21.04.1999
(73) Patentinhaber: Ivoclar Vivadent AG, 9494 Schaan (LI)
(72) Erfinder: Moszner, Norbert, Prof. Dr., 9492 Eschen (LI); Zeuner, Frank, Dr., 9490 Vaduz (LI); Rheinberger, Volker, Dr., 9490 Vaduz (LI)
(74) Vertreter: UEXKÜLL & STOLBERG

(56) Entgegenhaltungen:
- EP-A- 0 431 740
- GB-A- 2 089 807
- KAWAMOTO A M ET AL: "Novel class of difluorovinylphosphonate analogs of PEP" J. CHEM. SOC., PERKIN TRANS. 1 (JCPRB4,0300922X);97; (8); PP.1249-1253, - 21. April 1997 XP002089313 Space Activity Institute;Chemistry Division; Sao Jose dos Campos; Brazil (BR)
- CHEMICAL ABSTRACTS, vol. 117, no. 9, 31. August 1992 Columbus, Ohio, US; abstract no. 090506, IKEMURA K ET AL: "Preparation of carboxyalkylphosphonic acid and (meth)acrylic diesters as adhesive compositions" XP002089314 & JP 03 294286 A (MATSUKAZE K. K.) 25. Dezember 1991

## Beschreibung

Die vorliegende Erfindung betrifft polymerisierbare Acrylphosphonsäuren, die eine hohe Hydrolysestabilität aufweisen und sich insbesondere zur Herstellung oder als Bestandteile von Polymeren, Adhäsiven oder anderen Materialien und hauptsächlich von Dentalmaterialien eignen.

Polymerisierbare Phosphonsäuren sind vor allem als Comonomere von polymer-chemischer Bedeutung, und sie gestatten die Herstellung von organischen Polymeren, bei denen thermische Stabilität, Hafteigenschaften, Entflammbarkeit und Löslichkeit in polaren Lösungsmitteln verbessert sind. Für diesen Zweck sind zahlreiche monomere Phosphonsäuren mit polymerisierbaren Vinyl-, Dienyl-, Allyl- oder Styrylgruppen synthetisiert und polymerisiert worden. Eine Übersicht zu Phosphonsäuren gibt Houben-Weyl, Methoden oder Organischen Chemie, Band E 20 (2. Teil), Georg Thieme Verlag, Stuttgart-New York 1987, S. 1300 ff. Beispiele für solche konventionellen polymerisierbaren Phosphonsäuren sind Vinylphosphonsäure, Allylbenzolphosphonsäure, α-Aminoallylphosphonsäure, Phenylethenphosphonsäure, 1,3-Butadien- oder Isoprenphosphonsäure, 4-Vinylbenzolphosphonsäure oder 2-(4-Vinylphenyl)-ethanphosphonsäure.

Dabei zeigen Phosphonsäuren, bei denen die Doppelbindung direkt oder über ein Sauerstoffatom an das Phosphoratom gebunden ist, wie z.B. Vinylphosphonsäure oder Ethylphosphonsäuremonovinylester, allerdings eine nur mäßige Neigung zur Homopolymerisation. Demnach können aus ihnen nur Homopolymere mit einer geringen Molmasse erhalten werden. Hochmolekulare Polymerisate können demgegenüber von (Meth)acrylphosphonsäuren oder -estern erhalten werden, bei denen die (Meth)acrylgruppe nicht direkt am Phosphor gebunden ist. Bekannte (Meth)acrylphosphonsäure-Derivate sind z.B. die in der DE-B-27 11 234 beschriebenen Phenylphosphonsäuremono-(methacryloyloxyethyl)-ester der Formel (a) oder tert.-Butylphosphonsäuremono[1,3-di(methacryloyloxy)propan-2-yl)-ester der Formel (b).

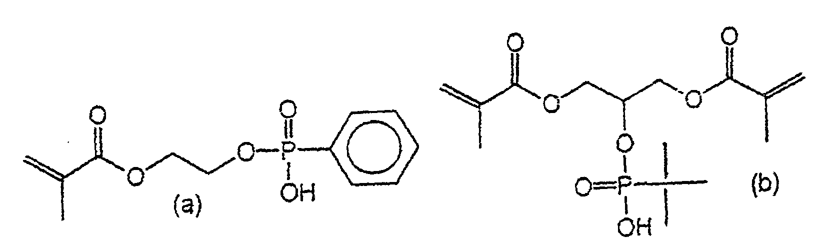

Darüber hinaus sind aus der DE-A-32 10 775 Acrylsäure-(2-phosphono-1,1-dimethylethylamin) und aus der DE-A-33 13 819 sowie der JP 62-63314 (Chem. Abstr. 107 (1987), 41318f) Methacrylsäure-(2-phosphono-1,1-dimethylethylamin) der Formel (c) bekannt.

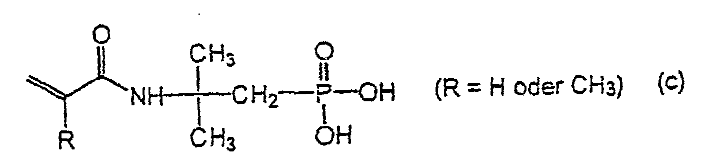

Acrylsäure-(2-phosphono-1,1-dimethylethylamin), auch als Acrylamido-2-methylpropanphosphonsäure bezeichnet, wird in Form ihrer Homo- oder Copolymeren als Korrosionsinhibitoren eingesetzt (vgl. EP-B-89 654 und US-A-4 650 591).

Kawamoto et. al. beschreiben im J. Chem. Soc., Perkin Trans. 1 (JCPRB4,0300922X); 97; (8) 1249-1253 die Synthese von 4,4-Difluor-4-phoshono-2-methylenbutansäure und -methylester, um Verbindungen zur Verfügung zu stellen, die die Synthese des Enzyms 5-Enolpyruvyl-shikimat-3-phosphat-Synthase inhibieren können.

Schließlich ist in der DD-A-273 846 auch N-Acryl-aminomethanbisphosphonsäure der Formel (d) beschrieben.

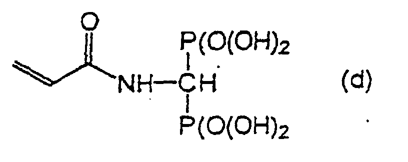

Allerdings sind alle diese bekannten (Meth)acrylphosphonsäure-Derivate) in wäßriger Lösung nicht stabil. Vielmehr tritt bei ihnen eine hydrolytische Abspaltung der (Meth)acrylgruppe auf, die durch dissozierte Protonen der Phosphonsäuregruppe sogar noch katalysiert und damit beschleunigt wird.

Bei einer ganzen Reihe von Anwendungen polymerisierbarer Phosphonsäuren ist aber der Einsatz von wäßrigen Lösungen von Vorteil oder zwingend notwendig. Dies ist z.B. der Fall bei der Herstellung von niedrigviskosen Adhäsiven, die frei von organischen Lösungsmitteln sind, oder bei dentalen Adhäsiven, die nur in wäßriger Form zu einer optimalen Benetzung der feuchten Dentinoberfläche führen.

Der Erfindung liegt demgemäß die Aufgabe zugrunde, polymerisierbare Acrylphosphonsäuren bereitzustellen, die in wäßriger Lösung hydrolysestabil sind sowie über gute Hafteigenschaften verfügen, mit herkömmlichen radikalischen Initiatoren polymerisiert werden können und sich daher als Bestandteil von insbesondere Adhäsiven, Formkörpern, Zementen oder Kompositen und vor allem von Dentalmaterialien eignen.

Diese Aufgabe wird überraschenderweise durch die hydrolysestabilen und polymerisierbaren Acryphosphonsäuren nach den Ansprüchen 1 und 2 gelöst.

Gegenstand der vorliegenden Erfindung sind ebenfalls das Verfahren zur Herstellung der Acrylphosphonsäuren nach Anspruch 3, deren Verwendung nach den Ansprüchen 4 bis 6, das Dentalmaterial nach den Ansprüchen 7 und 8 sowie Polymere und Copolymere der Acrylphosphonsäuren nach Anspruch 9.

Die erfindungsgemäßen Acrylphosphonsäuren sind Verbindungen der folgenden allgemeinen Formel (I), Stereoisomere davon und Gemische solcher Stereoisomeren 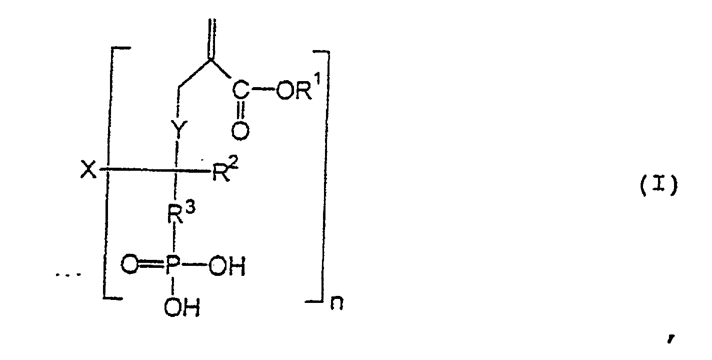 wobei R¹, R², R³, X, Y und n, sofern nicht anders angegeben, unabhängig voneinander die folgenden Bedeutungen haben:
- R¹ =: Wasserstoff, C₁- bis C₁₀-Alkyl oder C₆- bis C₁₀-Aryl,
- R² =: Wasserstoff, Fluor, C₁- bis C₅-Alkyl oder Phenyl,
- R³ =: C₁- bis C₈-Alkylen, Phenylen oder entfällt,
- Y =: Sauerstoff, Schwefel, C₁- bis C₈-Alkylen oder entfällt,
- n =: 1 oder 2,
und mit der Maßgabe, daß
(a) für n = 1
   - X =: Wasserstoff, Fluor, C₁- bis C₅-Alkyl oder C₆- bis C₁₂-Aryl,
   und
(b) für n = 2
   - X =: C₁- bis C₁₀-Alkylen, C₆- bis C₁₀-Arylen, C₇- bis C₂₀-Arylenalkylen oder entfällt.

Die einzelnen Alkyl- und Alkylenreste können dabei geradkettig, verzweigt oder cyclisch sein. Außerdem können die einzelnen Alkyl-, Aryl-, Alkylen-, Arylen-, Phenyl-, Phenylen- und Arylenalkylenreste einen oder mehrere Substituenten, wie Cl, Br, CH₃, C₂H₅, CH₃O, OH, COOH, CN oder NO₂, tragen.

Alleinig für die Zwecke des Anspruchs 1 sind die Verbindungen der Formel I ausgenommen, bei denen R¹ H oder C₁-Alkyl ist, R² Fluor ist, R³ entfällt, X Fluor ist, Y entfällt und n 1 ist.

Weiter existieren für die oben angegebenen Variablen der Formel (I) bevorzugte Definitionen, die, sofern nicht anders angegeben, unabhängig voneinander gewählt werden können und wie folgt sind:
- R¹ =: Wasserstoff, C₁- bis C₅-Alkyl oder Phenyl,
- R² =: Wasserstoff, Fluor oder C₁- bis C₃-Alkyl,
- R³ =: C₁- bis C₃-Alkylen, Phenylen oder entfällt,
- Y =: Sauerstoff, C₁- bis C₃-Alkylen oder entfällt,
- n =: 1 oder 2,
und mit der Maßgabe, daß
(a) für n = 1
   - X =: Wasserstoff, Fluor, C₁- bis C₃-Alkyl oder Phenyl,
   und
(b) für n = 2
   - X =: C₁- bis C₆-Alkylen, Phenylen oder entfällt.

Bevorzugte Verbindungen sind demgemäß solche, bei denen mindestens eine der variablen der Formel (I) die vorstehend beschriebene bevorzugte Definition aufweist, wobei die Formel (I) alle durch die genannten Substituenten möglichen Stereoisomere und ihre Mischungen, wie Racemate, einschließt.

Die erfindungsgemäßen Acrylphosphonsäuren der Formel (I) lassen sich durch Umsetzung von α-Halogenmethylacrylsäureestern der Formel (II) mit geschützten mono- oder difunktionellen Phosphonsäureestern der Formel (III) und Abspaltung der Schutzgruppen herstellen, wobei in den Formeln (II) und (III) U = Halogen, SG = Schutzgruppe und die übrigen Variablen so wie oben für Formel (I) definiert sind. Diese Umsetzung kann durch die folgende allgemeine Reaktionsgleichung veranschaulicht werden, der ein konkretes Beispiel folgt.

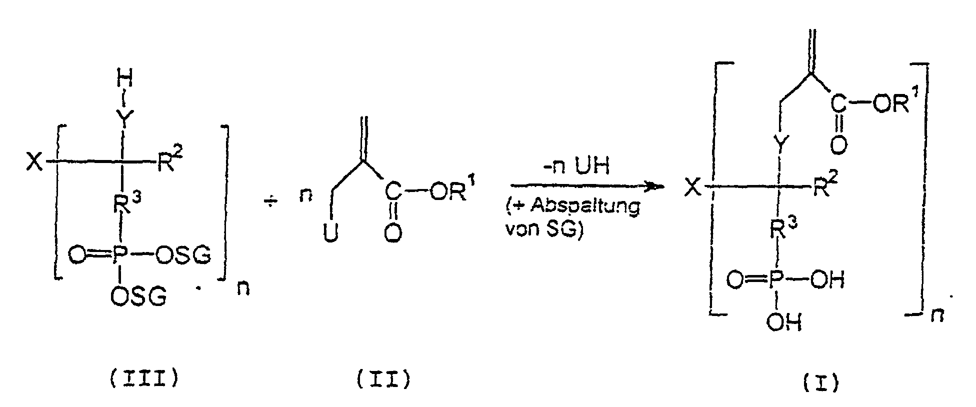

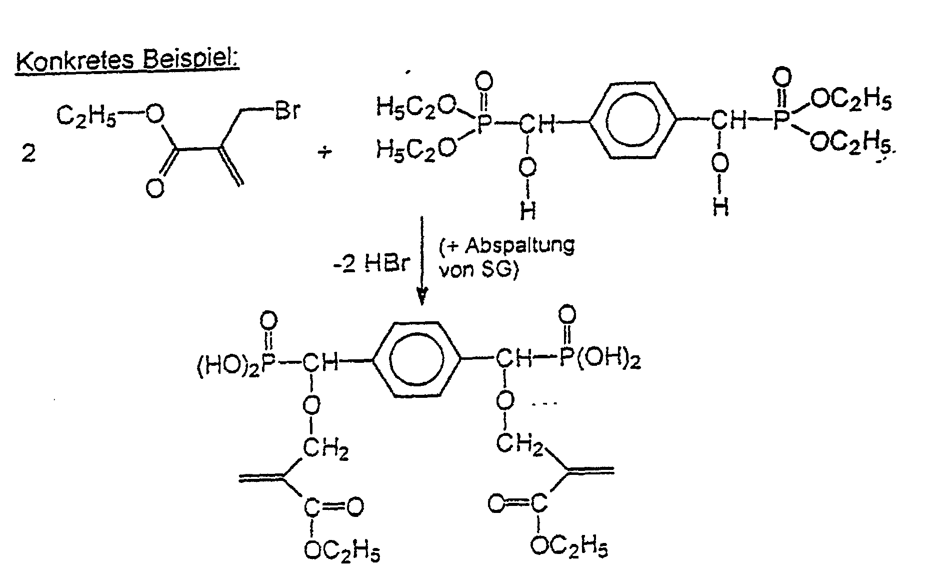

Dabei kann die Umsetzung unter Anwendung der aus der organischen Chemie bekannten Methoden für die Knüpfung von C-C-, C-O- oder C-S-Bindungen (vgl. C. Weygand, G. Hilgetag, Organisch-chemische Experimentierkunst, Johann Ambrosius Bart Verlag, Leipzig 1970, Seiten 963 ff., 362 ff. und 657 ff.) erfolgen.

Als Schutzgruppen (SG) werden für Phosphonsäuregruppen übliche Schutzgruppen, wie Estergruppen, insbesondere SG = Ethyl eingesetzt. Nach erfolgter Umsetzung werden diese entsprechend konventioneller Verfahren abgespalten, um die Acrylphosphonsäuren der Formel (I) freizusetzen. Die hydrolytische Abspaltung der Schutzgruppen SG erfolgt dann insbesondere durch Silylierung mit Trialkylsilanen, z.B. Trimethylsilylchlorid in Mischung mit Natriumiodid oder -bromid, und nachfolgende Umsetzung mit Alkoholen oder Wasser (vgl. S. Freeman, J. Chem. Soc., Perkin Trans, 2 (1991) 263).

Die als Ausgangsmaterialien eingesetzten α-Halogenmethylacrylsäureester (II) sind z.B. durch Reaktion von den entsprechenden Acrylsäureestern mit Formaldehyd in Gegenwart von 1,4-Diazabicyclo[2,2,2]octan (DABCO) und anschließender Halogenierung mit anorganischen Säurechloriden, wie SOCl₂, PCl₃ oder PBr₃, zugänglich (vgl. L.J. Mathias et al., Macromolecules 20 (1987) 2039, 2326, J. Polym. Sci.: Part A: Polym. Chem. 32 (1994) 2937), und diese Reaktion wird durch die folgende Gleichung und ein konkretes Beispiel veranschaulicht:

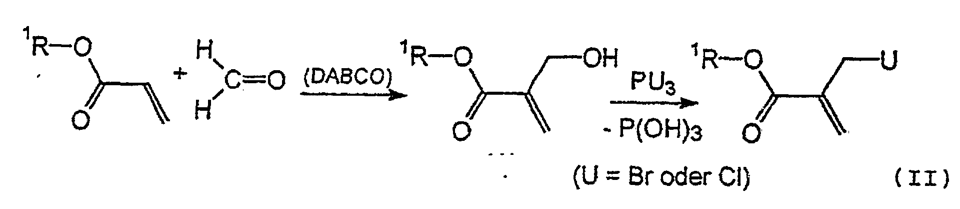

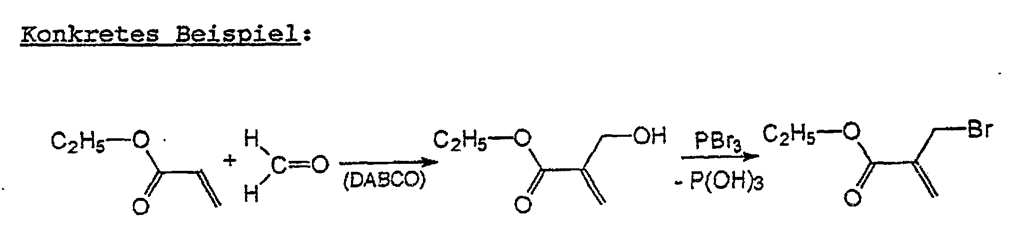

Geeignete geschützte mono- oder difunktionelle Phosphonsäureester (III) können auf unterschiedlichen Wegen erhalten werden. Ein besonders geeigneter Weg verläuft über die Michaelis-Arbusow-Reaktion zur Herstellung von Alkylphosphonsäureestern (vgl. G.M. Kosolapoff, Org. Reactions 6 (1951) 273). Dabei werden Trialkylphosphite, z.B. Triethylphosphit, und Halogenalkane entsprechen nachstehender GLeichungen zur Reaktion gebracht, wobei gegebenenfalls auch die Y-H-Gruppe geschützt werden muß.

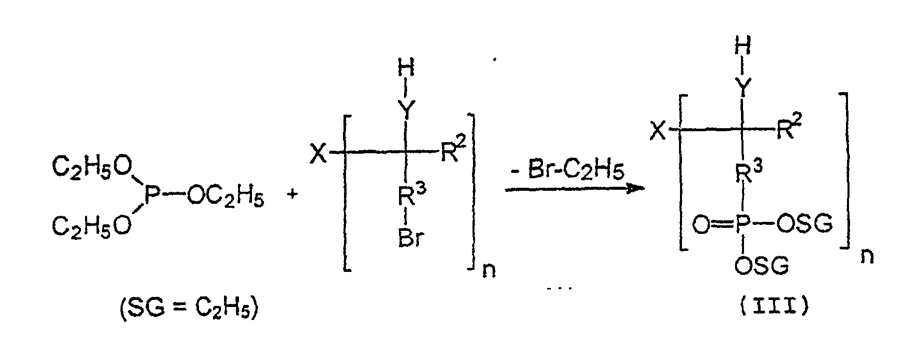

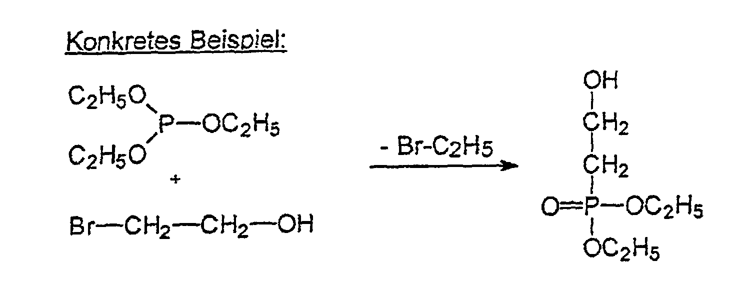

Arylphosphonsäuren sind z.B. durch Friedel-Crafts-Reaktion von aromatischen Kohlenwasserstoffen mit Phosphortrichlorid in Gegenwart von Aluminiumtrichlorid, Chlorierung des gebildeten Dichlorphosphins zum Tetrachlorphosphin und anschließender Hydrolyse zur Phosphonsäure zugänglich (vgl. G.M. Kosolapoff, Org. Reactions 6 (1951) 273).

Weiter können geschützte Hydroxyalkylphosphonsäureester (Y-H = OH) mit R³ = entfällt in der Weise hergestellt werden, daß Dialkylphosphite unter Basenkatalyse an mono- oder difunktionelle Aldehyde oder Ketone analog des Verfahrens nach F. Texier-Boullet, A. Foucaud, Synthesis, 1982, 916 angelagert werden. Diesen Reaktionstyp sowie ein konkretes Beispiel dafür zeigen die folgenden Reaktionsgleichungen:

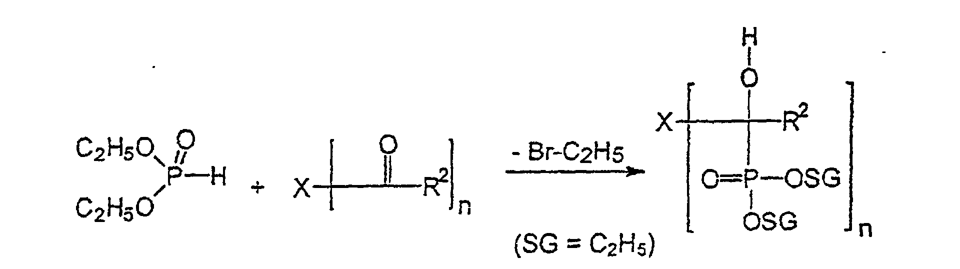

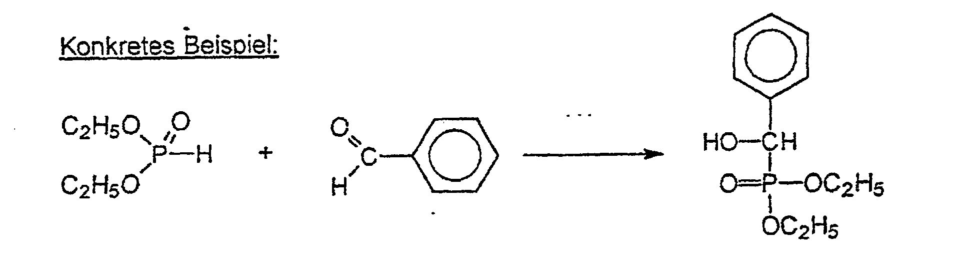

Beispiele für die erfindungsgemäßen Acrylphosphonsäuren der Formel (I) sind u.a.:

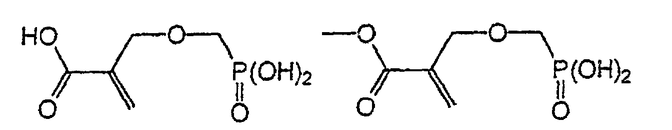

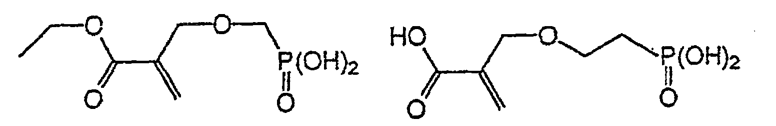

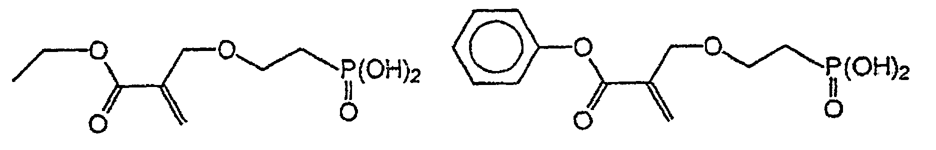

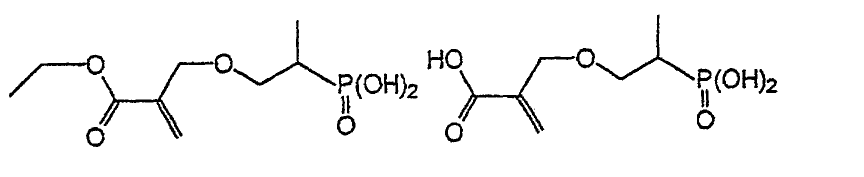

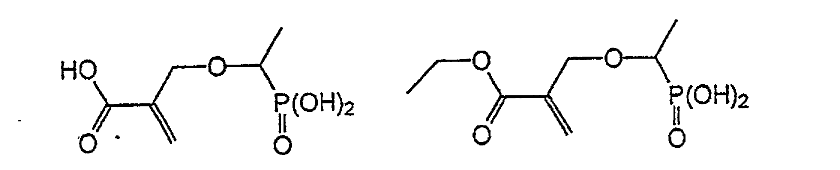

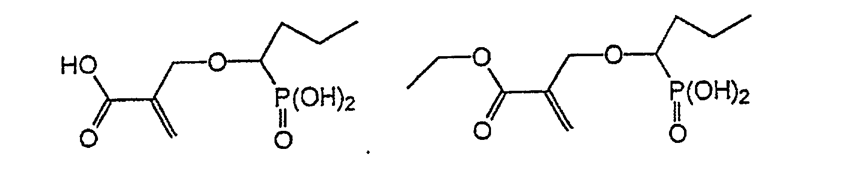

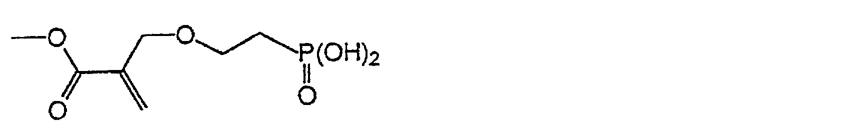

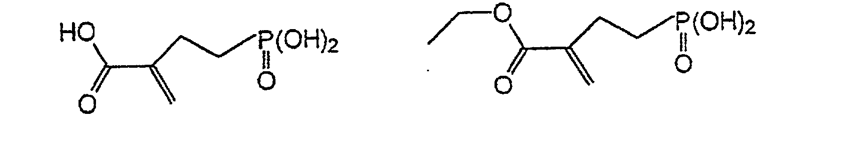

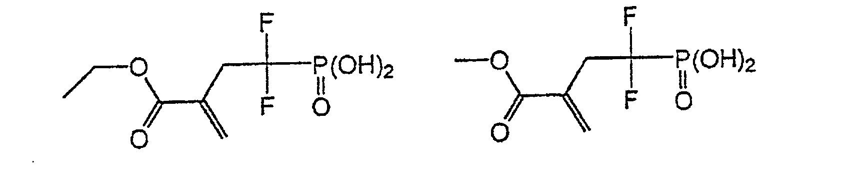

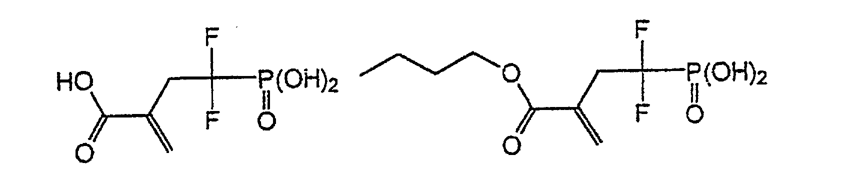

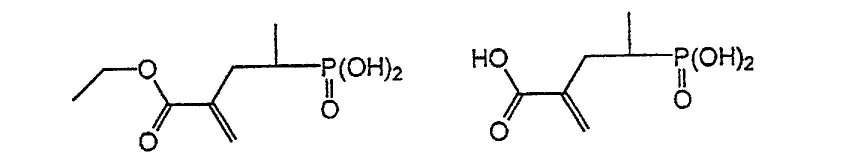

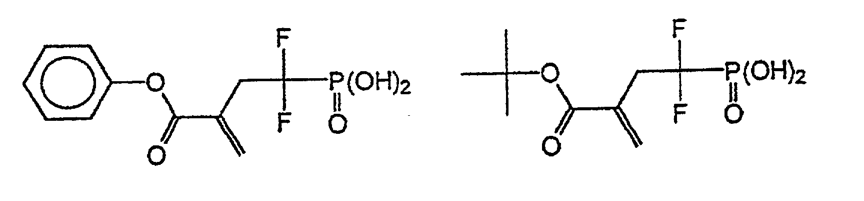

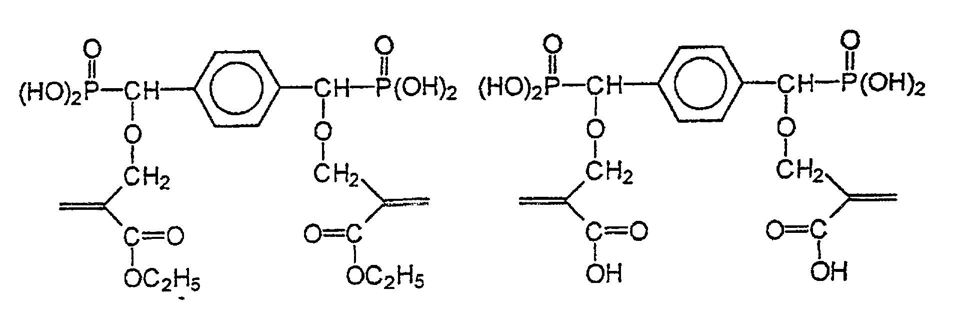

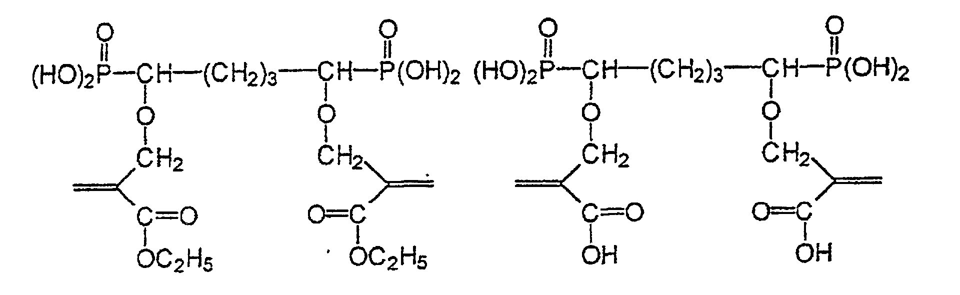

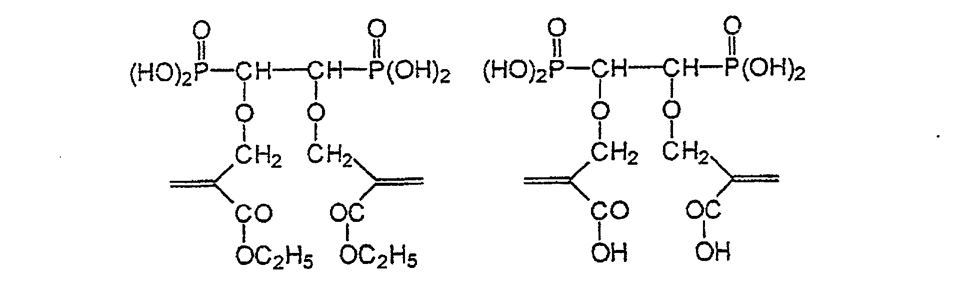

Aufgrund des Vorliegens von polymerisierbaren Gruppen eignen sich die erfindungsgemäßen Acrylphosphonsäuren als Ausgangsmaterialien für die Herstellung von Polymeren und Copolymeren. Dabei lassen sie sich mit den bekannten Methoden der radikalischen Polymerisation homopolymerisieren oder z.B. mit geeigneten Comonomeren copolymerisieren.

Zur Durchführung der Polymerisation können die bekannten radikalischen Initiatoren (vgl. Encyclopedia of Polymer Science and Engineering, Vol. 13, Wiley-Interscience Publisher, New York 1988, 754 ff.) eingesetzt werden. Es eignen sich besonders Azoverbindungen, wie Azobis(isobutyronitril) (AIBN) oder Azobis-(4-cyanvaleriansäure) oder Peroxide, wie Dibenzoylperoxid, Dilauroylperoxid, tert.-Butylperoctoat, tert.-Butylperbenzoat oder Di-(tert.-butyl)-peroxid.

Als Initiatoren für die Heißhärtung eignen sich auch Benzpinakol und 2,2'-Dialkylbenzpinakole.

Weiterhin können auch Photoinitiatoren (vgl. J.P. Fouassier, J.F. Rabek (Hrsg.), Radiation Curing in Polymer Science and Technology, Vol. II, Elsevier Applied Science, London und New York 1993) für die Polymerisation mit UV-Licht oder Licht sichtbarer Wellenlängen, wie Benzoinether, Dialkylbenzilketale, Dialkoxyacetophenone, Acylphosphinoxide, α-Diketone, wie 9,10-Phenanthrenchinon, Diacetyl, Furil, Anisil, 4,4'-Dichlorbenzil und 4,4'-Dialkoxybenzil, und Campherchinon, verwendet werden.

Die Acrylphosphonsäuren können insbesondere als Bestandteil von Adhäsiven, Zementen, Kompositen und Formkörpern sowie bevorzugt von Dentalmaterialien verwendet werden. Dabei ist es möglich, daß sie in zumindest teilweise polymerisierter Form vorliegen. Weitere Komponenten, mit denen die Acrylphosphonsäuren kombiniert werden können, sind nachstehend erwähnt.

Die erfindungsgemäßen Acrylphosphonsäuren lassen sich allein oder in Mischung mit herkömmlichen radikalisch polymerisierbaren Comonomeren, insbesondere mit difunktionellen Vernetzermonomeren polymerisieren. Für die Herstellung von Adhäsiven oder Dentalmaterialien eignen sich vor allem vernetzende bi- oder mehrfunktionelle Acrylate oder Methacrylate, wie z.B. Bisphenol-Adi(meth)acrylat, als Bis-GMA bezeichnetes Additionsprodukt von Methacrylsäure und Bisphenol-A-diglycidylether, als UDMA bezeichnetes Additionsprodukt von Hydroxyethylmethacrylat und 2,2,4-Trimethylhexamethylendiisocyanat, Di-, Tri- oder Tetraethylenglykoldi(meth)acrylat, Decandioldi(meth)acrylat, Trimethylolpropantri(meth)acrylat und Pentaerythrittetra(meth)acrylat. Es eignen sich ebenfalls die durch Veresterung von (Meth)acrylsäure mit den entsprechenden Diolen zugänglichen Verbindungen Butandioldi(meth)acrylat, 1,10-Decandioldi(meth)acrylat und 1,12-Dodecandioldi(meth)acrylat.

Darüber hinaus können die erfindungsgemäßen Acrylphosphonsäuren oder ihre Mischungen mit anderen radikalisch polymerisierbaren Comonomeren zur Verbesserung der mechanischen Eigenschaften mit organischen oder anorganischen Partikeln oder Fasern gefüllt werden. Bevorzugte anorganische partikuläre Füllstoffe sind amorphe kugelförmige Materialien auf der Basis von Mischoxiden aus SiO₂, ZrO₂ und/oder TiO₂, mikrofeine Füllstoffe, wie pyrogene Kieselsäure oder Fällungskieselsäure, sowie Makro- oder Minifüllstoffe, wie Quarz-, Glaskeramik- oder Glaspulver mit einer durchschnittlichen Teilchengröße von 0,01 bis 5 µm. Schließlich können auch röntgenopake Füllstoffe, wie Ytterbiumtrifluorid, oder Glasfasern, Polyamid- oder Kohlenstoff-Fasern eingesetzt werden.

Den Acrylphosphonsäuren können im Bedarfsfall weitere Komponenten zugesetzt werden, vor allem Lösungsmittel, wie Wasser, Ethylacetat, Aceton, Ethanol oder Mischungen von diesen, sowie Stabilisatoren, UV-Absorber, Farbstoffe, Pigmente oder Gleitmittel.

Die erfindungsgemäßen Acrylphosphonsäuren eignen sich besonders als Bestandteil von Dentalmaterialien, wie Befestigungszemente und Füllungskomposite und vor allem Dentaladhäsive. Solche Materialien zeichnen sich durch eine sehr gute Haftung auf unterschiedlichen Substraten, wie der Zahnhartsubstanz und metallischen Substraten, aus, was auf die eingesetzten Acrylphosphonsäuren zurückgeführt werden kann. Es wird angenommen, daß die Acrylphosphonsäuren ionische und/oder Komplexverbindungen mit den Calcium-Ionen der Zahnhartsubstanz oder den Metallionen von metallischen Substraten ausbilden. Diese führen zu einer höheren Haftung als es aufgrund einfacher Dipol-Dipol- oder van der Waals-Wechselwirkung möglich wäre.

Die überraschend hohe Hydrolysestabilität der Acrylphosphonsäuren verleiht dabei den erfindungsgemäßen Materialien ebenfalls eine sehr gute Hydrolysestabilität. Das gilt sowohl für das unpolymerisierte als auch das polymerisierte Material. Eine hohe Hydrolysestabilität ist naturgemäß für solche Materialien von besonderer Bedeutung, die permanent wäßrigen Medien ausgesetzt sind, wie dies gerade bei Dentalmaterialien der Fall ist, die für einen längere Verbleib in der Mundhöhle vorgesehen sind.

Bevorzugte erfindungsgemäße Dentalmaterialien enthalten die folgenden Komponenten (a), (b), (c), (d) und/oder (e):
(a) 1 bis 99 Gew.-%, bevorzugt 10 bis 80 Gew.-% und besonders bevorzugt 20 bis 70 Gew.-% erfindungsgemäße Acrylphosphonsäuren,
(b) 0,01 bis 5 Gew.-% und bevorzugt 0,1 bis 2,0 Gew.-% radikalischer Initiator,
(c) 0 bis 80 Gew.-%, bevorzugt 0 bis 60 Gew.-% und besonders bevorzugt 0 bis 50 Gew.-% radikalisch polymerisierbare Comonomere,
(d) 0 bis 95 Gew.-%, bevorzugt 0 bis 80 Gew.-% und besonders bevorzugt 0 bis 70 Gew.-% Lösungsmittel,
(e) 0 bis 90 Gew.-%, besonders bevorzugt in Abhängigkeit von der Anwendung 0 bis 20 Gew.-% (Adhäsiv), 20 bis 60 Gew.-% (Zement) und 60 bis 85 Gew.-% (Füllungskomposit) Füllstoff.

Die Erfindung wird im folgenden anhand von Beispielen näher erläutert.

### Beispiele

### Beispiel 1

### 1. Stufe: 2-[3-(Diethoxyphosphoryl)-2-oxa-propyl]-acrylsäureethylester (1)

Zu einer Lösung von 8,4 g (50 mmol) Hydroxymethylphosphonsäurediethylester, der einfach durch Umsetzung von Diethylphosphit mit Paraformaldehyd zugänglich ist, 5,05 g (50 mmol) Triethylamin (TEA) und 0,01 g Phenothiazin (Stabilisator) in 40 ml absolutem Tetrahydrofuran (THF) wurden bei Raumtemperatur unter Rühren 7,45 g (50 mmol) α-Chlormethylacrylsäureethylester zugegeben. Nach 30 Minuten Rühren bei Raumtemperatur wurde 16 Stunden unter Rückfluß erwärmt. Nach Abkühlen auf Raumtemperatur wurde der gebildete Niederschlag an Triethylammoniumchlorid abfiltriert. Das Filtrat wurde mit 150 ml Wasser verdünnt, mit 2 N Salzsäure auf einen pH-Wert von ca. 5 bis 7 eingestellt und mehrfach mit Diethylether extrahiert. Nach Trocknen über wasserfreiem Na₂SO₄ wurde der Extrakt im Rotationsverdampfer eingeengt, im Feinvakuum getrocknet und schließlich fraktioniert destilliert. Man erhielt 10,8 g [Kp.: 120-125 °C (0,007 mbar)] einer farblosen Flüssigkeit (77 % Ausbeute).

| Elementaranalyse: | | | |
|---|---|---|---|
| C₁₁H₂₁O₆P (280,26) | Ber. | C 47,14 | H 7,55 |
| | Gef. | C 47,58 | H 7,87 |

IR (KBr, cm⁻¹): 780 (w), 818 (w), 877 (w), 967 (s), 1029 (s,sh), 1112 (s), 1176 (m), 1261 (s), 1306 (m), 1392 (m,sh), 1446 (w), 1719 (s), 2908 (w) und 2984 (m).

¹H-NMR (300 MHz, CDCl₃, ppm): 1,29-1,42 (m, 9H, CH₃), 3,80 (d, 2H, CH₂-P), 4,10-4,26 (m, 6H, CH₂-CH₃), 4,34 (s, 2H,CH₂-C=CH₂), 5,90 und 6,33 (s, 2×1H, C=CH₂).

¹³C-NMR (75 MHz, CDCl₃, ppm): 14,20 und 16,47 (CH₃↓), 60,78, 62,50, 63,68, 65,89 und 71,38 (alle CH₂↑), 126,66 (CH₂=C↑), 136,64 (CH₂=C(-)) und 165,51 (C=O(-)).

³¹P-NMR (121,5 MHz, CDCl₃, ppm): 43,0.

### 2. Stufe: 2-[3-(Dihydroxyphosphoryl)-oxa-propyl]-acrylsäureethylester (2)

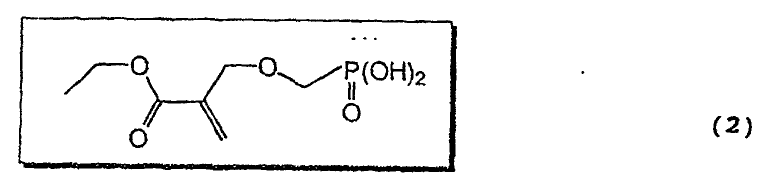

Zu einer Lösung von 14,0 g (50 mmol) der Verbindung (1) und 0,01 g Hydrochinonmonomethylether (MEHQ, Stabilisator) in 30 ml absolutem Methylenchlorid wurden 16,8 g (110 mmol) Trimethylsilylbromid zugetropft, und die Mischung wurde 90 Minuten unter Rückfluß gerührt. Danach wurde im Rotationsverdampfer eingeengt, und der erhaltene Rückstand wurde nach Aufnahme in 50 ml Methanol 2 Stunden gerührt. Nach Behandeln der leicht rötlichen Lösung mit Aktivkohle wurde im Vakuum eingeengt und anschließend im Feinvakuum bis zur Gewichtskonstanz getrocknet. Es blieben 9,1 g (81 % Ausbeute) eines viskosen Öls zurück, das eine mittels HPLC bestimmte Reinheit von 98 % aufwies.

| Elementaranalyse: | | | |
|---|---|---|---|
| C₇H₁₃O₆P (224,15) | Ber. | C 37,51 | H 5,85 |
| | Gef. | C 37,26 | H 6,87 |

IR (KBr, cm⁻¹): 684 (w), 778 (w), 820 (m), 861 (m), 970 (s), 1020 (s), 1112 (s), 1182 (s,sh), 1309 (s,sh), 1405 (m,sh), 1466 (m,sh), 1632 (m), 1713 (s), 2324 (b) und 2600-3500 (b).

¹H-NMR (300 MHz, Aceton-d₆, ppm): 0,32 (ca 1 % Silylverbindung), 1,28 (t, 3H, CH₃), 3,86 (d, 2H, PCH₂), 4,20 (q, 2H, CH₂CH₃), 4,33 (s, 2H, CH₂C=C), 5,96 und 6,30 (s, 2×1H, C=CH₂) und 11,38 (s, b, 2H, OH).

¹³C-NMR (75 MHz, Aceton-d₆, ppm): 14,2 (CH₃↓), 61,25 (CH₂CH₃↑), 64,75 und 66,9 (d, CH₂P↑), 71,35 (CH₂C=C↑), 126,15 (C=CH₂↑), 137,6 (C=CH₂(-)) und 165,75 (C=O).

³¹P-NMR (121,5 MHz, Aceton-d₆, ppm): 47,0

### Beispiel 2

### 1. Stufe: 2-[4-Dimethoxyphosphoryl)-2-oxa-butyl]-acrylsäureethylester (3)

Zu einer Lösung von 7,7 g (50 mmol) Hydroxyethylphosphonsäuredimetyhlester, 5,05 g (50 mmol) TEA und 0,01 g Phenothiazin in 40 ml absolutem THF wurden bei Raumtemperatur unter Rühren 7,45 g (50 mmol) α-Chlormethylacrylsäureethylester zugegeben. Es wurde dann analog zu Beispiel 1 (1. Stufe) weiter verfahren. Die fraktionierte Destillation ergab 2,1 g [Kp.: 115-120°C (0,005 mbar)] einer farblosen Flüssigkeit (16 % Ausbeute).

| Elementaranalyse: | | | |
|---|---|---|---|
| C₁₀H₁₉O₆P (266,23) | Ber. | C 45,11 | H 7,19 |
| | Gef. | C 45,45 | H 7,26 |

IR (KBr, cm⁻¹): 645 (w), 732 (m), 820 (s), 954 (m), 1032 (s,b), 1105 (s), 1179 (s), 1267 (s), 1306 (s), 1375 (m,sh), 1464 (m,sh), 1640 (m), 1715 (s), 2234 (w), 2956 (m,sh) und 3472 (w,b).

¹H-NMR (300 MHz, CDCl₃, ppm): 1,31 (t, 3H, CH₃CH₂), 2,10-2,21 (m, 2H, CH₂P), 3,71-3,83 (m, 8H, 2×CH₃O + CH₂CH₂O), 4,18-4,28 (m, 4H, CH₃CH₂O + CH₂C=), 5,89 und 6,29 (s, 2×1H, C=CH₂).

¹³C-NMR (75 MHz, CDCl₃, ppm): 14,2 (CH₃CH₂↓), 24,0 und 27,0 (CH₂P↑), 52,3 (CH₃O), 60,95 (CH₃CH₂↑), 64,6 (CH₂CH₂O↑), 69,0 (OCH₂C=C↑), 126,0 (C=CH₂↑), 137,4 (C=CH₂(-)) und 165,6 (C=O(-)).

³¹P-NMR (121,5 MHz, CDCl₃, ppm): 62,0

### 2. Stufe: 2-[4-Dihydroxyphosphoryl)-2-oxa-butyl]-acrylsäureethylester (4)

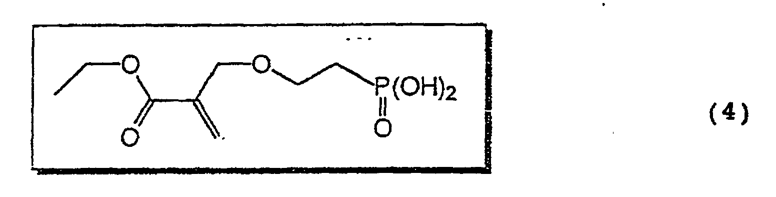

Analog zu Beispiel 1 (2. Stufe) wurden zu einer Lösung von 6,1 g (23 mmol) der Verbindung (3) und 0,01 g MEHQ in 20 ml absolutem Methylenchlorid 7,7 g (50 mmol) Trimethylsilylbromid zugetropft. Es wurde weiter 90 Minuten unter Rückfluß gerührt, danach eingeengt, der Rückstand mit 30 ml Methanol versetzt und entsprechend Beispiel 1 (2. Stufe) aufgearbeitet. Nach Trocknen im Feinvakuum bis zur Gewichtskonstanz wurden 4,3 g (79 % Ausbeute) eines viskosen Öls als Produkt erhalten.

| Elementaranalyse: | | | |
|---|---|---|---|
| C₈H₁₅O₆P (238,18) | Ber. | C 40,34 | H 6,35 |
| | Gef. | C 40,86 | H 6,52 |

IR (KBr, cm⁻¹): 718 (w), 820 (w), 1024 (s,sh), 1103 (s), 1178 (s,sh), 1273 (m,sh), 1307 (m), 1376 (m,sh), 1466 (w,sh), 1637 (m), 1717 (s), 2323 (b) und 2800-3300 (m,b).

¹H-NMR (300 MHz, Aceton-d₆, ppm): 1,28 (t, 3H, CH₃), 2,07-2,24 (m, 2H, CH₂P), 3,72-3,84 (m, 2H, CH₂CH₂O), 4,15-4,25 (m, 4H, CH₃CH₂O + CH₂C=C), 5,93 und 6,25 (s, 2×1H, CH₂=) und 9,80 (s, 2H, OH).

¹³C-NMR (75 MHz, Aceton-d₆, ppm): 13,0 (CH₃), 27,8 und 30,3 (d, CH₂P), 61,4 (CH₂CH₃), 65,58 (CH₂CH₂O), 71,6 (CH₂C=CH₂), 125,7 (CH₂C=CH₂), 138,5 (CH₂C=CH₂) und 166,1 (C=O).

³¹P-NMR (121,5 MHz, CDCl₃, ppm): 60,0.

### Beispiel 3

### 1. Stufe: 1,4-Bis[1-(diethoxyphosphoryl)-1-hydroxymethyl]-benzol (5)

Zu einer Lösung von 13,4 g (0,1 mol) Terephthalaldehyd und 29,0 g (0,2 mol) Diethylphosphit in 50 ml absolutem Acetonitril wurden 1,1 g (0,01 mol) DABCO bei ca. 15 °C unter Rühren zugegeben. Nach weiterem Rühren über Nacht wurde der gebildete Niederschlag abgesaugt, jeweils mit etwas Acetonitril und Petrolether gewaschen und bis zur Gewichtskonstanz getrocknet. Es wurden 30,7 g (75 % Ausbeute) eines weißen Feststoffes (Smp.: 195-200 °C) erhalten.

| Elementaranalyse: | | | |
|---|---|---|---|
| C₁₆H₂₈O₈P₂ (410,34) | Ber. | C 46,83 | H 6,68 |
| | Gef. | C 46,79 | H 6,43 |

IR (KBr, cm⁻¹): 445 (w), 494 (w), 575 (s), 658 (w), 758 (m), 791 (w), 831 (w), 861 (w), 975 (s), 1022 (s), 1056 (s), 1205 (s), 1230 (s), 1392 (m), 1445 (w), 1478 (w), 1509 (w), 1702 (w), 2911 (w), 2988 (m) und 3263 (s,b).

¹H-NMR (300 MHz, DMSO-d₆, ppm): 1,12-1,18 (m, 12H, CH₃), 3,82-3,98 (m, 8H, CH₂), 4,92 (d, 2H, CH-P), 6,20 (s, 2H, OH, H/D-Austausch) und 7,38 (s, 4H, CH-arom.).

¹³C-NMR (100 MHz, DMSO-d₆, ppm): 16,13 und 16,23 (s, CH₃), 61,68 und 62,08 (s, CH₂), 68,31 und 69,93 (d, CH-P), 126,68 (s, CH-arom.) und 137,54 (s, quart. arom.C).

³¹P-NMR (162 MHz, DMSO-d₆, ppm): 21,9.

### 2. Stufe: 1,4-Bis[1-(diethoxyphosphoryl)-1-[(2-methylen-3-yl-propansäureethylester)-oxy]methyl]-benzol (6)

Zu einer Lösung von 12,3 g (0,03 mol)-der Verbindung (5), 6,05 g (60 mmol) TEA und 0,02 g Phenothiazin in 100 ml absolutem THF wurden bei Raumtemperatur unter Rühren 8,9 g (60 mmol) α-Chlormethylacrylsäureethylester zugegeben. Nach 30 Minuten Rühren bei Raumtemperatur wurde 16 Stunden unter Rückfluß erwärmt. Nach Abkühlen auf Raumtemperatur wurde der gebildete Niederschlag abfiltriert, einmal mit Diethylether und zweimal mit Wasser gewaschen. Nach Trocknen des Rückstandes bei 60°C im Feinvakuum wurden 9,7 g (51 % Ausbeute) eines farblosen Harzes erhalten.

| Elementaranalyse: | | | |
|---|---|---|---|
| C₂₈H₄₄O₁₂P₂ (634,60) | Ber. | C 52,98 | H 6,99 |
| | Gef. | C 52,98 | H 7,08 |

IR (KBr, cm⁻¹): 580 (s), 751 (m), 795 (m), 859 (w), 967 (s), 1028 (s), 1055 (s), 1095 (s), 1177 (s), 1257 (s), 1308 (s,sh), 1391 (s,sh), 1445 (m,sh), 1508 (w), 1636 (m), 1716 (s), 2930 (m,sh) und 2982 (s).

¹N-NMR (400 MHz, Aceton-d₆, ppm): 1,10-1,22 (m, 18H, CH₃), 3,95-4,01 (m, 8H, POCH₂CH₃), 4,10-4,16 (m, 8H, COCH₂), 4,95 (d, 2H, CH), 5,97 und 6,27 (s, 2×2H, CH₂=) und 7,43 (s, 4H, CH-arom.).

¹³C-NMR (100 MHz, Aceton-d₆, ppm): 14,16 (CH₃-Methacrylat), 16,37 (CH₃-Phosphonat), 60,73 (OCH₂CH₃-Methacrylat), 62,9 und 63,2 (d, OCH₂CH₃-Phosphonat), 68,70 und 68,83 (d, CH-P), 126,82 und 128,54 (CH₂= und CH-arom.), 134,97 und 136,75 (CH₂=C und C-arom.) und 165,37 (C=O).

³¹P-NMR (121,5 MHz, DMSO-d₆, ppm): 18,6.

### 3. Stufe: 1,4-Bis[1-(dihydroxyphosphoryl)-1-[(2-methylen-3-yl-propansäureethylester)oxy]methyl]-benzol (7)

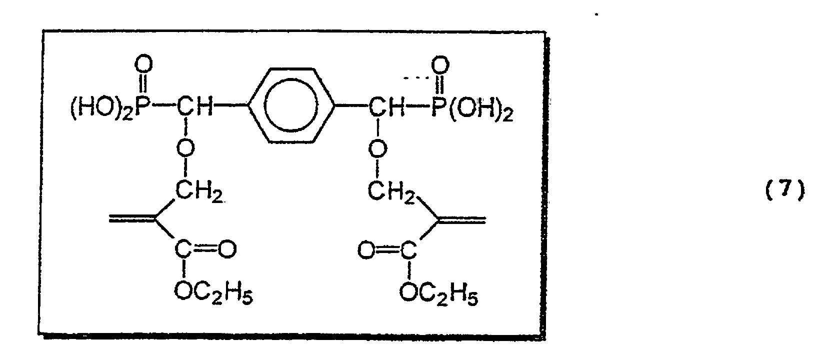

Zu einer Lösung von 7,8 g (12,3 mmol) der Verbindung (6) und 0,01 g MEHQ in 20 ml absolutem Methylenchlorid wurden 8,6 g (56 mmol) Trimethylsilylbromid zugetropft, und die erhaltene Mischung wurde 75 Minuten unter Rückfluß gerührt. Danach wurde am Rotationsverdampfer eingeengt. Nach Zugabe von 20 ml Methanol zu dem Rückstand wurde über Nacht gerührt und erneut im Vakuum eingeengt. Das gebildete hellgelbe Pulver wurde in 100 ml einer gesättigten NaHCO₃-Lösung aufgenommen und mit zweimal je 50 ml Methylenchlorid gewaschen. Dann wurde die Lösung mit Aktivkohle verrührt und filtriert. Das Filtrat wurde mit konz. Salzsäure auf pH = 1 eingestellt, mit 2 g NaCl und 50 ml Wasser versetzt und anschließend dreimal mit je 150 ml Methylenchlorid ausgeschüttelt. Die vereinigten Extrakte wurden über Na₂SO₄ getrocknet und am Rotationsverdampfer bis zur Trockene eingeengt. Der Rückstand wurde im Feinvakuum bis zur Gewichtskonstanz getrocknet. Es blieben 4,3 g (66 % Ausbeute) eines schwach gelblichen kristallinen Produktes zurück.

| Elementaranalyse: | | | |
|---|---|---|---|
| C₂₀H₂₈O₁₂P₂ (522,39) | Ber. | C 45,99 | H 5,45 |
| | Gef. | C 44,96 | H 4,93 |

IR (KBr, cm⁻¹): 412 (m), 569 (s), 646 (w), 815 (m), 941 (s), 1035 (s), 1093 (s), 1176 (s), 1285 (sh), 1320 (sh), 1390 (sh), 1406 (m), 1460 (w), 1509 (w), 1636 (m), 1717 (s), 2987 (s) und 3440 (s,b).

¹H-NMR (300 MHz, DMSO-d₆/CDCl₃, ppm): 1,17 (t, 6H, CH₃), 4,12-4,33 (m, 8H, CH₂), 4,50-4,55 (d, 2H, CH), 6,08 und 6,27 (s, 2×1H, =CH₂), 7,36 (s, 4H, CH-arom.) und 9-10 (4H, b, OH).

¹³C-NMR (100 MHz, DMSO-d₆/CDCl₃, ppm): 14,01 (CH₃), 60,37 (OCH₂CH₃), 68,10 und 68,23 (d, CH-P), 125,87 und 127,49 (CH₂= und CH-arom.), 135,64 und 136,85 (CH₂=C und C-arom.) und 165,34 (C=O).

³¹P-NMR (121,5 MHz, DMSO-d₆, ppm): 15,1.

### Beispiel 4

### 1. Stufe: [(Dimethoxyphosphoryl)-[(2-methylen-3-yl-propansäureethylester)oxy]-methyl]benzol (8)

Zu einer Lösung von 21,6 g (0,1 mol) [(Dimethoxyphosphoryl)-1-hydroxymethyl]benzol, das durch Umsetzung von Dimethylphosphit mit Benzaldehyd zugänglich ist (F. Texier-Boullet, A. Foucaud, Synthesis 1982, 916), 10,1 g (0,1 mol) TEA und 0,02 g Phenothiazin in 200 ml absolutem THF wurden bei Raumtemperatur unter Rühren 14,9 g (0,1 mol) α-Chlormethylacrylsäureethylester zugegeben. Nach 30 Minuten Rühren bei Raumtemperatur wurde 16 Stunden unter Rückfluß erwärmt. Nach Abkühlen auf Raumtemperatur wurde der gebildeten Niederschlag abfiltriert, und dieser wurde einmal mit Diethylether gewaschen. Der Waschether und das Filtrat wurden mit 400 ml Wasser verdünnt, und die erhaltene Mischung wurde dreimal mit jeweils 100 ml Ether extrahiert. Die vereinigten organischen Extrakte wurden mit 100 ml gesättigter NaCl-Lösung gewaschen, über Na₂SO₄ getrocknet und am Rotationsverdampfer eingeengt. Der zurückgebliebene ölige Rückstand wurde dann im Hochvakuum destilliert. Es wurden 15,9 g (49 % Ausbeute) einer farblosen Flüssigkeit [Kp.: 153-155 °C (10⁻⁵ mbar)] erhalten.

| Elementaranalyse: | | | |
|---|---|---|---|
| C₁₅H₂₁O₆P (328,30) | Ber. | C 54,88 | H 6,45 |
| | Gef. | C 54,10 | H 6,24 |

IR (KBr, cm⁻¹): 465 (s,b), 701 (m), 771 (w), 832 (m), 1031 (s,sh), 1095 (s), 1181 (s), 1262 (s), 1309 (m), 1401 (w), 1453 (m), 1637 (w), 1718 (s), 2854 (w) und 2956 (m).

¹N-NMR (300 MHz, CDCl₃, ppm): 1,29 (t, 3H, CH₃CH₂), 3,65-3,73 (dd, 6H, POCH₃), 4,14-4,33 (m, 4H, CH₂), 4,78 (d, 1H, CH), 5,97 und 6,34 (s, 2×1H, CH₂=) und 7,34-7,47 (m, 5H, CH-arom.).

¹³C-NMR (100 MHz, CDCl₃, ppm): 14,45 (s, CH₃-Methacrylat↑); 54,17 (d, CH₃-OP↑); 61,07 (OCH₂CH₃-Methacrylat↓); 69,88 (s, OCH₂C=CH₂↓); 77,34 und 79,31 (d, CH-P↑); 126,87 (s, CH₂=↓); 128,32 und 128,86 (s, CH-arom.↑); 134,97 und 136,75 (CH₂=C und C-arom.(-)); 165,37 (C=O(-)).

³¹P-NMR (121,5 MHz, CDCl₃, ppm): 21,3 (s).

### 2. Stufe: 1-(Dihydroxyphosphoryl)-1-[(2-methylen-3-ylpropionsäureethylester)-oxy]methyl]-benzol (9)

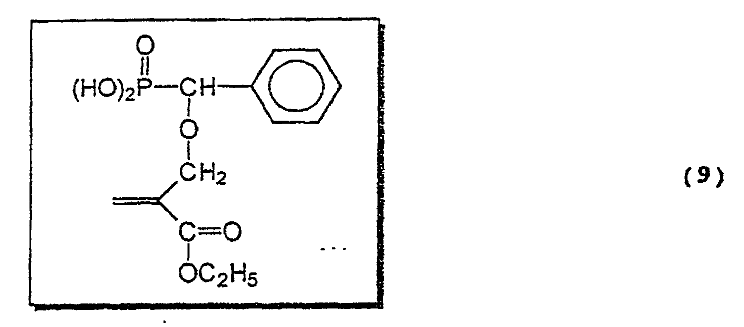

Zu einer Lösung von 14,4 g (44 mmol) der Verbindung (8) und 0,01 g MEHQ in 40 ml absolutem Methylenchlorid wurden 17,2 g (0,1 mol) Trimethylsilylbromid zugetropft, und die erhaltenen Mischung wurde 75 Minuten unter Rückfluß gerührt. Es wurde dann am Rotationsverdampfer eingeengt und der Rückstand mit 40 ml Methanol versetzt. Die Mischung wurde 4 Stunden gerührt im Vakuum eingeengt. Das gebildete Harz wurde in 200 ml einer gesättigten NaHCO₃-Lösung aufgenommen und zweimal mit 100 ml Methylenchlorid gewaschen. Die Lösung wurde mit Aktivkohle verrührt und filtriert. Das Filtrat wurde danach mit konz. Salzsäure auf pH = 1 eingestellt, mit 4 g NaCl und 50 ml Wasser versetzt und anschließend dreimal mit je 100 ml Methylenchlorid ausgeschüttelt. Die vereinigten Extrakte wurden über Na₂SO₄ getrocknet und am Rotationsverdampfer bis zur Trockene eingeengt. Der Rückstand wurde im Feinvakuum bis zur Gewichtskonstanz getrocknet. Es wurden 10,0 g (76 % Ausbeute) eines klebrigen kristallinen Produktes erhalten.

| Elementaranalyse: | | | |
|---|---|---|---|
| C₁₃H₁₇O₆P (300,25) | Ber. | C 52,00 | H 5,71 |
| | Gef. | C 50,47 | H 5,58 |

IR (KBr, cm⁻¹): 698 (s), 739 (w), 805 (w), 819 (w), 970 (s), 1026 (s,sh), 1094 (s,sh), 1178 (s,sh), 1280 (s,sh), 1320 (m), 1340 (m), 1402 (m,sh), 1453 (m,sh), 1490 (m), 1643 (m), 1713 (s), 2321 (m), 2910 (m), 2949 (m) und 2982 (m).

¹H-NMR (300 MHz, CDCl₃, ppm): 1,22 (t, 3H, CH₃), 4,06-4,17 (m, 4H, CH₂), 4,50 (d, 2H, CH-P), 5,89 und 6,19 (s, 2×1H, CH₂=), 7,18-7,33 (m, 5H, CH-arom.) und 10,79 (s, 2H, OH, H/D-Aust.).

¹³C-NMR (100 MHz, CDCl₃, ppm): 14,08 (CH₃); 60,86 (OCH₂CH₃); 68,49 (CH₂OCH); 77,20 und 78,86 (d, CH-P); 127,49-128,25, 135,21, 136,15 (alle C-arom. + CH₂=C) und 166,08 (C=O).

³¹P-NMR (121,5 MHz, CDCl₃, ppm): 20,1.

### Beispiel 5

### Radikalische Homopolymerisation der Acrylphosphonsäure (7)

2,61 g (5,0 mmol) der als Monomer eingesetzten Acrylphosphonsäure (7) und 2,5 Mol-% Azobis(isobutyronitril), bezogen auf Monomer, wurden in 9,0 ml Ethanol in einem Schlenkgefäß gelöst. Die Monomerlösung wurde durch mehrfach wiederholtes Einfrieren unter Argon und Auftauen unter Feinvakuum entgast und anschließend bei 65 °C unter Argon polymerisiert. Aufgrund der difunktionellen Monomerstruktur fiel bereits nach 2 Minuten vernetzte und damit unlösliche polymere Acrylphosphonsäure (7) aus. Der Monomerumsatz betrug nach 1 Stunde 47,7 %. Das gebildete Polymer war unlöslich.

### Beispiel 6

### Untersuchung der hydrolytischen Stabilität der monomeren Acrylphosphonsäuren

Von den monomeren erfindungsgemäßen Acrylphosphonsäuren (2) und (7) sowie als Vergleichsbeispiel monomerer 2-(Methacryloyloxy)ethylphosphonsäure (10) wurden jeweils 20-Gew.%ige Lösungen in EtOD/H₂O (1 : 1-Volumenanteile) hergestellt und bei 25 und 37 °C gelagert. Zur Bestimmung der hydrolytischen Stabilität wurde nach verschiedenen Zeiten ein ¹H-NMR-Spektrum aufgenommen, und dieses wurde auf die Bildung von möglichen Spaltprodukten untersucht.

Dabei zeigte sich, daß bei den erfindungsgemäßen Acrylphosphonsäuren (2) und (7) selbst nach 3 Monaten keine hydrolytische Abspaltung der polymerisierbaren Gruppe erfolgt war, während im Falle der herkömmlichen Acryphosphonsäure (10) bei 25 °C schon nach wenigen Stunden die Abspaltung der Ethylphosphonsäuregruppe begann und sich durch das Vorliegen von 2-Hydroxyethylphosphonsäure als Spaltprodukt nachweisen ließ. Das läßt auf eine hydrolytische Spaltung entsprechend nachstehender Formel schließen.

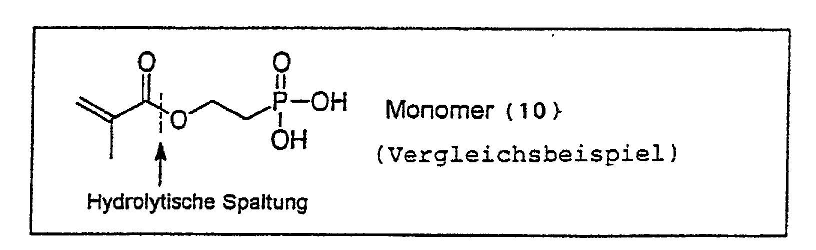

### Beispiel 7

### Untersuchung der Metallhaftung der Acrylphosphonsäuren

Eine handelsübliche Ni-Cr-Mo-Dentallegierung Wiron 88 (Thyssen-Bego) wurde sandgestrahlt und mit Heißdampf gereinigt. Anschließend wurde ein Primer aus 10,0 Gew.% der Acrylphosphonsäure (7), 45,0 Gew.% Wasser, 44,7 Gew.% Ethanol und 0,3 Gew.% Campherchinon in dünner Schicht aufgepinselt, und es wurde darauf Variolink (Vivadent Ets., Liechtenstein), ein kommerzielles lichthärtendes Adhäsiv für Füllungskomposite, aufgetragen und belichtet. Danach wurde eine teilbare Teflonform (d=4mm, h=6mm) mit einer Halterung auf der Metalloberfläche fixiert, und es wurde ein lichthärtendes Füllungskomposit, nämlich Tetric (Vivadent Ets., Liechtenstein), in einem durch die Teflonform vorgegebenen Volumen und einer dadurch bestimmten Haftfläche schichtenweise auf die Metallfläche aufpolymerisiert. Die Scherhaftwerte wurden nach 24 Stunden Lagerung in Wasser bei 37°C gemäß ISO-TR 11405 bestimmt. Die im Scherversuch ermittelte Haftfestigkeit ergab einen ausgezeichneten Wert von 11,6±2,0 MPa.

## Patentansprüche

1. Hydrolysestabile und polymerisierbare Acrylphosphonsäuren der allgemeinen Formel (I), Stereoisomere davon und Mischungen von diesen 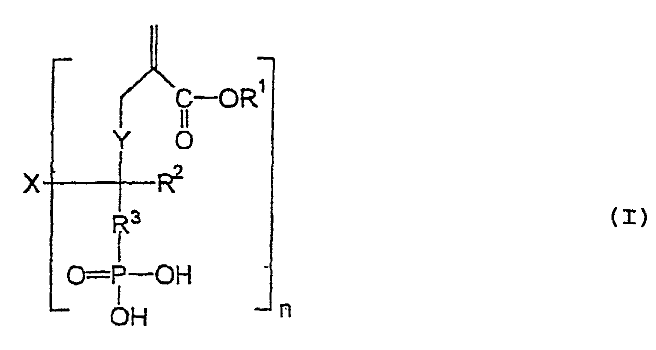 wobei R¹, R², R³, X, Y und n, sofern nicht anders angegeben, unabhängig voneinander die folgenden Bedeutungen haben:
R¹ = Wasserstoff, C₁- bis C₁₀-Alkyl oder C₆- bis C₁₀-Aryl,
R² = Wasserstoff, Fluor, C₁- bis C₅-Alkyl oder Phenyl,
R³ = C₁- bis C₈-Alkylen, Phenylen oder entfällt,
Y = Sauerstoff, Schwefel, C₁- bis C₈-Alkylen oder entfällt,
n = 1 oder 2,
und mit der Maßgabe, daß
(a) für n = 1
X = Wasserstoff, Fluor, C₁- bis C₅-Alkyl oder C₆- bis C₁₂-Aryl,
und
(b) für n = 2
X = C₁- bis C₁₀-Alkylen, C₆- bis C₁₀-Arylen, C₇- bis C₂₀-Arylenalkylen oder entfällt,
und wobei die einzelnen Alkyl-, Aryl-, Alkylen-, Arylen-, Phenyl-, Phenylen- und Arylenalkylenreste einen oder mehrere Substituenten tragen können und wobei eine Verbindung der Formel (I) ausgenommen ist, bei der R¹, R², R³, X, Y und n die folgenden Bedeutungen haben:
R¹= Wasserstoff oder C₁-Alkyl,
R²= Fluor,
R³= entfällt,
X= Fluor,
Y= entfällt und
n= 1.

2. Acrylphosphonsäuren nach Anspruch 1, **dadurch gekennzeichnet, daß** die Variablen der Formel (I), sofern nicht anders angegeben, unabhängig voneinander die folgenden Bedeutungen haben:
R¹ = Wasserstoff, C₁- bis C₅-Alkyl oder Phenyl,
R² = Wasserstoff, Fluor oder C₁- bis C₃-Alkyl,
R³ = C₁- bis C₈-Alkylen, Phenylen oder entfällt,
Y = Sauerstoff, C₁- bis C₈-Alkylen oder entfällt,
n = 1 oder 2,
und mit der Maßgabe, daß
(a) für n = 1
X = Wasserstoff, Fluor, C₁- bis C₅-Alkyl oder Phenyl,
und
(b) für n = 2
X = C₁- bis C₆-Alkylen, Phenylen oder entfällt.

3. Verfahren zur Herstellung von Acrylphosphonsäuren der allgemeinen Formel (I), Stereoisomeren davon und Mischungen von diesen 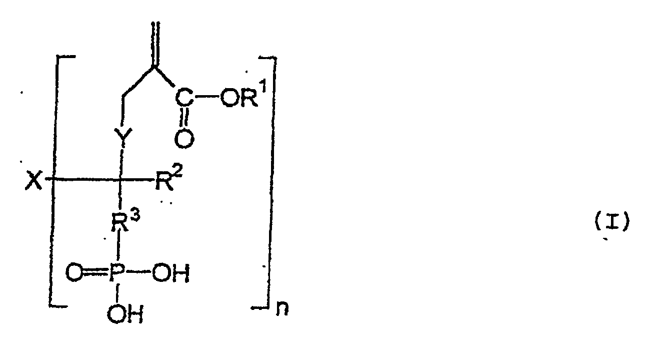 wobei R¹, R², R³, X, Y und n, sofern nicht anders angegeben, unabhängig voneinander die folgenden Bedeutungen haben:
R¹ = Wasserstoff, C₁- bis C₁₀-Alkyl oder C₆- bis C₁₀-Aryl,
R² = Wasserstoff, Fluor, C₁- bis C₅-Alkyl oder Phenyl,
R³ = C₁- bis C₈-Alkylen, Phenylen oder entfällt,
Y = Sauerstoff, Schwefel, C₁- bis C₈-Alkylen oder entfällt,
n = 1 oder 2,
und mit der Maßgabe, daß
(a) für n = 1
X = Wasserstoff, Fluor, C₁- bis C₅-Alkyl oder C₆- bis C₁₂-Aryl,
und
(b) für n = 2
X = C₁- bis C₁₀-Alkylen, C₆- bis C₁₀-Arylen, C₇- bis C₂₀-Arylenalkylen oder entfällt,
und wobei die einzelnen Alkyl-, Aryl-, Alkylen-, Arylen-, Phenyl-, Phenylen- und Arylenalkylenreste einen oder mehrere Substituenten tragen können, **dadurch gekennzeichnet, daß** man α-Halogenmethylacrylsäureester der allgemeinen Formel (II) 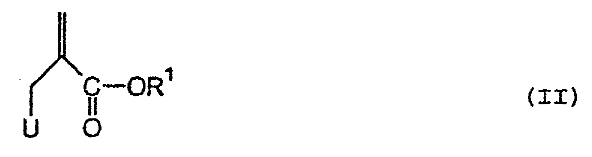 mit geschützten mono- oder difunktionellen Phosphonsäureestern der allgemeinen Formel (III) 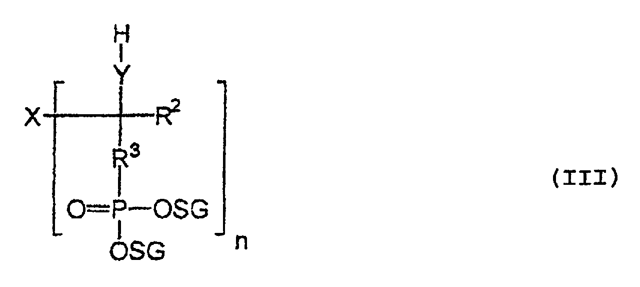 umsetzt und die Schutzgruppen abspaltet, wobei in den Formeln (II) und (III)
U = Halogen und
SG = Schutzgruppe
sind.

4. Verwendung der in Anspruch 3 definierten Acrylphosphonsäuren als Bestandteil eines Adhäsivs, eines Polymeren, eines Komposits, eines Zements, eines Formkörpers und insbesondere eines Dentalmaterials.

5. Verwendung nach Anspruch 4, **dadurch gekennzeichnet, daß** das Dentalmaterial ein Dentaladhäsiv, ein Befestigungszement oder ein Füllungskomposit ist.

6. Verwendung nach Anspruch 4 oder 5, **dadurch gekennzeichnet, daß** die Acrylphosphonsäuren in zumindest teilweise polymerisierter Form vorliegen.

7. Dentalmaterial, **dadurch gekennzeichnet, daß** es die in Anspruch 3 definierte Acrylphosphonsäure enthält.

8. Dentalmaterial nach Anspruch 7, **dadurch gekennzeichnet, daß** es die Acrylphosphonsäure in zumindest teilweise polymerisierter Form enthält.

9. Polymere und Copolymere, **dadurch gekennzeichnet, daß** sie durch Polymerisation oder Copolymerisation der in Anspruch 3 definierten Acrylphosphonsäuren erhältlich sind.

## Claims

1. Hydrolysis-stable and polymerisable acrylophosphonic acids of the general formula (I), stereoisomers thereof and mixtures of these 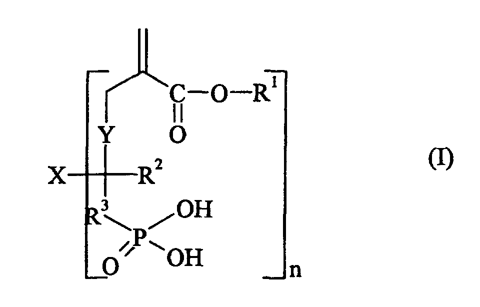 where R¹, R², R³, X, Y and n, unless stated otherwise, independently of one another have the following meanings:
R¹ = hydrogen, C₁ to C₁₀ alkyl or C₆ to C₁₀ aryl,
R² = hydrogen, fluorine, C₁ to C₅ alkyl or phenyl,
R³ = C₁ to C₈ alkylene, phenylene or is absent,
Y = oxygen, sulphur, C₁ to C₈ alkylene or is absent,
n = 1 or 2,
and with the proviso that
(a) for n = 1
X = hydrogen, fluorine, C₁ to C₅ alkyl or C₆ to C₁₂ aryl,
and
(b) for n = 2
X = C₁ to C₁₀ alkylene, C₆ to C₁₀ arylene, C₇ to C₂₀ arylenealkylene or is absent,
and wherein the individual alkyl, aryl, alkylene, arylene, phenyl, phenylene and arylenealkylene radicals can bear one or more substituents and wherein a compound of formula (I) is excluded when R¹, R², R³, X, Y and n have the following meanings:
R¹ = hydrogen or C₁ alkyl,
R² = fluorine,
R³ = absent,
X = fluorine,
Y = absent and
n = 1.

2. Acrylophosphonic acids according to Claim 1, wherein the variables of the formula (I), unless otherwise stated, independently of one another have the following meanings:
R¹ = hydrogen, C₁ to C₅ alkyl or phenyl,
R² = hydrogen, fluorine or C₁ to C₃ alkyl,
R³ = C₁ to C₃ alkylene, phenylene or is absent,
Y = oxygen, C₁ to C₃ alkylene or is absent,
n = 1 or 2,
and with the proviso that
(a) for n = 1
X = hydrogen, fluorine, C₁ to C₃ alkyl or phenyl,
and
(b) for n=2
X = C₁ to C₆ alkylene, phenylene or is absent.

3. Process for the preparation of acrylophosphonic acids of the general formula (I), stereoisomers thereof and mixtures of these 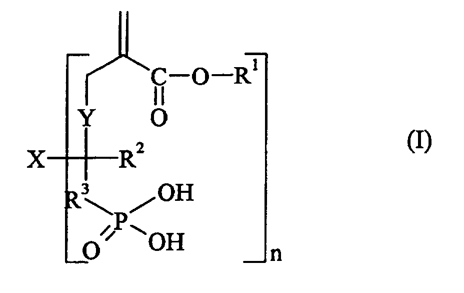 where R¹, R², R³, X, Y and n, unless stated otherwise, independently of one another have the following meanings:
R¹ = hydrogen, C₁ to C₁₀ alkyl or C₆ to C₁₀ aryl,
R² = hydrogen, fluorine, C₁ to C₅ alkyl or phenyl,
R³ = C₁ to C₈ alkylene, phenylene or is absent,
Y = oxygen, sulphur, C₁ to C₈ alkylene or is absent,
n= 1 or 2,
and with the proviso that
(a) for n = 1
X = hydrogen, fluorine, C₁ to C₅ alkyl or C₆ to C₁₂ aryl,
and
(b) for n = 2
X = C₁ to C₁₀ alkylene, C₆ to C₁₀ arylene, C₇ to C₂₀ arylenealkylene or is absent,
and wherein the individual alkyl, aryl, alkylene, arylene, phenyl, phenylene and arylenealkylene radicals can bear one or more substituents, **characterised in that** an α-halomethylacrylic acid ester of the general formula (II) is reacted 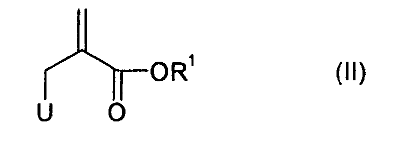 with protected mono or difunctional phosphonic acid esters of the general formula (III) 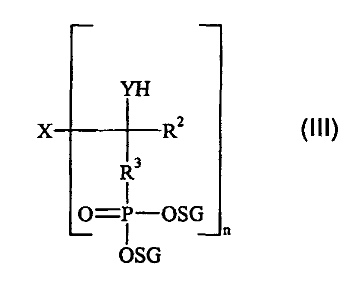 and the protecting groups are cleaved, whereby in the formulae (II) and (III)
U = halogen and
SG = protecting group.

4. Use of the acrylophosphonic acids defined in Claim 3 as a component of an adhesive, of a polymer, of a composite, of a cement, of a shaped body and in particular of a dental material.

5. Use according to Claim 4, **characterised in that** the dental material is a dental adhesive, a fixing cement or a filling composite.

6. Use according to Claim 4 or 5, **characterised in that** the acrylophosphonic acids are present in at least partially polymerised form.

7. Dental material, **characterised in that** it comprises the acrylphosphonic acid defined in Claim 3.

8. Dental material according to Claim 7, **characterised in that** it comprises the acrylophosphonic acid in at least partially polymerised form.

9. Polymers and copolymers, **characterised in that** they are obtainable by polymerisation or copolymerisation of the acrylophosphonic acids defined in Claim 3.

## Revendications

1. Acides acrylphosphoniques stables vis-à-vis de l'hydrolyse et polymérisables, de formule générale (I), leurs stéréoisomères ainsi que des mélanges de ceux-ci 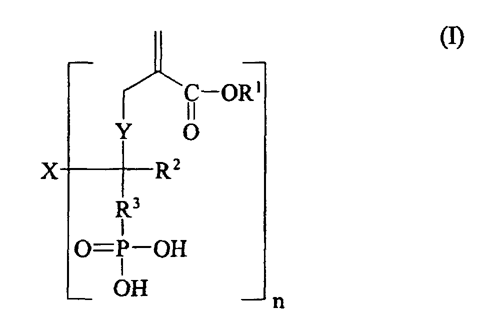 dans lesquels, sauf indication contraire, R¹, R², R³, X, Y et n ont indépendamment les uns des autres les significations suivantes :
R¹ = hydrogène, alkyle en C₁ à C₁₀ ou aryle en C₆ à C₁₀,
R² = hydrogène, fluor, alkyle en C₁ à C₅ ou phényle,
R³ = alkylène en C₁ à C₈, phénylène ou absent,
Y = oxygène, soufre, alkylène en C₁ à C₈ ou absent,
n = 1 ou 2,
et à la condition que
(a) pour n = 1
X = hydrogène, fluor, alkyle en C₁ à C₅ ou aryle en C₆ à C₁₂,
et
(b) pour n = 2
X = alkylène en C₁ à C₁₀, arylène en C₆ à C₁₀, arylènealkylène en C₇ à C₂₀ ou absent,
et dans laquelle les différents résidus alkyle, aryle, alkylène, arylène, phényle, phénylène et arylènealkylène peuvent porter un ou plusieurs substituants, à l'exception d'un composé de la formule (I) dans laquelle R¹, R², R³, X, Y et n ont les
R¹ = hydrogène ou alkyle en C₁,
R² = fluor,
R³ = absent,
X = fluor,
Y = absent et
n = 1.

2. Acides acrylphosphoniques selon la revendication 1, **caractérisés en ce que**, sauf indication contraire, les variables de la formule (I) ont indépendamment les unes des autres les significations suivantes :
R¹ = hydrogène, alkyle en C₁ à C₅ ou phényle,
R² = hydrogène, fluor ou alkyle en C₁ à C₃,
R³ = alkylène en C₁ à C₃, phénylène ou absent,
Y = oxygène, alkylène en C₁ à C₃ ou absent,
n = 1 ou 2,
et à la condition que
(a) pour n = 1
X = hydrogène, fluor, alkyle en C₁ à C₃ ou phényle,
et
(b) pour n = 2
X = alkylène en C₁ à C₆, phénylène ou absent.

3. Procédé de préparation d'acides acrylphosphoniques stables vis-à-vis de l'hydrolyse et polymérisables, de formule générale (I), de leurs stéréoisomères ainsi que de mélanges de ceux-ci 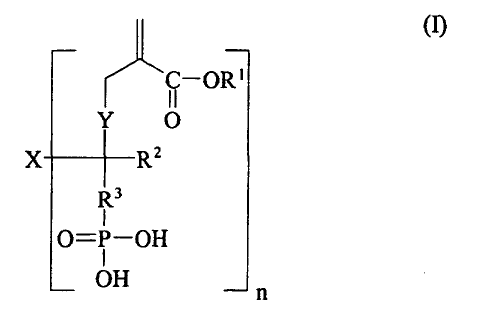 dans laquelle, sauf indication contraire, R¹, R², R³, X, Y et n ont indépendamment les uns des autres les significations suivantes :
R¹ = hydrogène, alkyle en C₁ à C₁₀ ou aryle en C₆ à C₁₀,
R² = hydrogène, fluor, alkyle en C₁ à C₅ ou phényle,
R³ = alkylène en C₁ à C₈, phénylène ou absent,
Y = oxygène, soufre, alkylène en C₁ à C₈ ou absent,
n = 1 ou 2,
et à la condition que
(a) pour n = 1
X = hydrogène, fluor, alkyle en C₁ à C₅ ou aryle en C₆ à C₁₂,
et
(b) pour n = 2
X = alkylène en C₁ à C₁₀, arylène en C₆ à C₁₀, arylènealkylène en C₇ à C₂₀ ou absent,
et dans laquelle les différents résidus alkyle, aryle, alkylène, arylène, phényle, phénylène et arylènealkylène peuvent porter un ou plusieurs substituants, **caractérisé en ce qu'**on fait réagir un ester d'acide α- halométhylacrylique de formule générale (II) 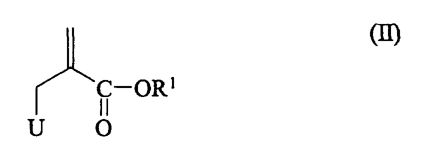 avec des esters d'acide phosphonique mono- ou difonctionnels protégés de formule générale (III) 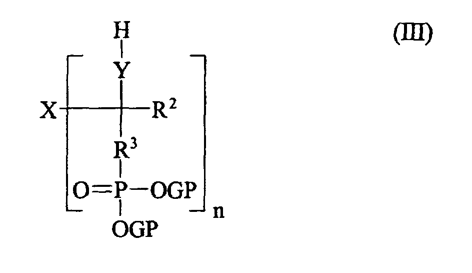 et on dissocie les groupes protecteurs, avec dans les formules (II) et (III)
U = halogène, et
GP = groupe protecteur.

4. Utilisation des acides acrylphosphoniques définis dans la revendication 3 à titre de constituant d'un adhésif, d'un polymère, d'un composite, d'un ciment, d'un élément moulé et en particulier d'un matériau dentaire.

5. Utilisation selon la revendication 4, **caractérisée en ce que** le matériau dentaire est un adhésif dentaire, un ciment de fixation ou un composite d'obturation.

6. Utilisation selon la revendication 4 ou la revendication 5, **caractérisée en ce que** les acides acrylphosphoniques sont présents sous une forme au moins partiellement polymérisée.

7. Matériau dentaire, **caractérisé en ce qu'**il contient l'acide acrylphosphonique défini dans la revendication 3.

8. Matériau dentaire selon la revendication 7, **caractérisé en ce qu'**il contient l'acide acrylphosphonique sous une forme au moins partiellement polymérisée.

9. Polymères et copolymères, **caractérisés en ce qu'**ils peuvent sont susceptibles d'être obtenus par polymérisation ou copolymérisation des acides acrylphosphoniques définis dans la revendication 3.
